# EUROPEAN PATENT APPLICATION

(11) **EP 2 735 302 A1**
(43) Date of publication of application: **28.05.2014**
(21) Application number: 12007881.1
(22) Date of filing: 22.11.2012
(51) Int. Cl.: A61K 8/49, A61Q 7/00, A61Q 7/02, A61Q 17/04, A61Q 19/08, A61K 8/97, C12C 3/08

(54) **Topical composition comprising a hop extract and containing 8-prenyl naringenin, and uses thereof**

(71) Applicant: KAIROSmetics UG, 23564 Lübeck (DE)
(72) Inventor: The designation of the inventor has not yet been filed
(74) Representative: Müller-Boré & Partner Patentanwälte PartG mbB

(57) **Abstract**

The present invention relates to a topical composition comprising a hop extract and containing 8-prenyl naringenin (8-PN), and uses of said composition.

## Description

The present invention relates to a topical composition comprising a hop extract and containing 8-prenyl naringenin (8-PN), and uses of said composition.

Hop is an old and well known cultural plant having its main relevance and use still in the production of beer. While in the past dried and minced whole hop was used for spicing the beer, nowadays extracts from hops are also used. However, some producers have not changed the process and are further using whole hops or a mixture of plant particles and hop extracts. Hop contains a number of secondary metabolites showing a variety of properties. The bitter acids and other aroma compounds are used for the spicing of beer. A slight sedative effect of hop is postulated in folk medicine, for which the oxidation-sensitive α- and ß-bitter acids seem to contribute. In addition, radical scavenger properties were reported for bitter acids.

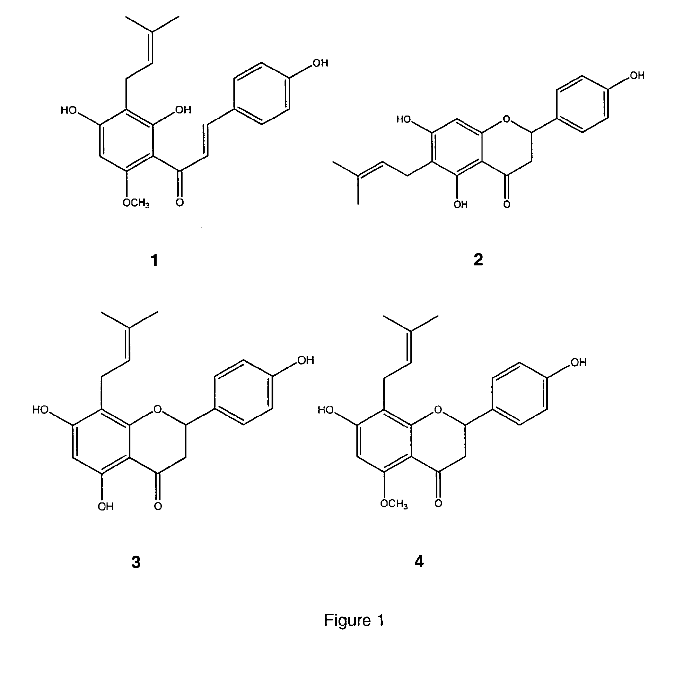

More recently, the phenolic compounds from hop were investigated. Besides the longer known isoprenylated chalcone xanthohumol (XH) (cf. Figure 1, compound **1**), the isoprenylated flavonoids 6-prenyl naringenin (6-PN) (cf. Figure 1, compound **2),** 8-prenyl naringenin (8-PN) (cf. Figure 1, compound 3) and iso-xanthohumol (IXH) (cf. Figure 1, compound **4)** were found and pharmacologically characterized. XH was shown to exaggerate a number of anti-carcinogenic activities *in vitro,* including radical scavenger, antiinflammatory, anti-estrogenic, and anti-proliferative effects. IXH, into which XH is isomerized during e.g. the brewing process of beer, shows similar qualitative effects as XH but at a much lower potency level. No such pronounced effects were reported for 6-PN, although prostate cell growth was inhibited by 50 % at rather high concentrations of 18 µM 6-PN.

A rather different pharmacological profile was found for 8-PN. In particular, 8-PN was reported to have a pronounced estrogenic activity. 8-PN is a pure estrogen receptor (ER) agonist with no hidden anti-estrogenic, androgenic or progestogenic activity. It is 2- to 3-fold more active at the ERα than at ERß. The potency of the 2S(-)-enantiomer of 8-PN is slightly higher than the 2R(+)-enantiomer, showing a similarly high activity as estrone, and having an about 70-fold lower activity than estradiol. Thus, 8-PN is the most potent phyto-estrogen known, superseding the estrogenic activity of genistein by a factor of 100. 8-PN was found to inhibit angiogenesis in the high nanomolar range, to induce tissue plasminogen activator at low micromolar concentrations, and to inhibit CYP1A2, the key enzyme in activating chemical carcinogenesis, in the low micromolar range. In contrast to naringenin, 8-PN did not inhibit CYP3A4, a key enzyme in metabolism of xenobiotics. In addition, 8-PN was able to inhibit copper induced protein oxidation in the high nanomolar range. The surprising tissue specificity of 8-PN (bone > uterus) found in juvenile rats was encouraging enough to administer chemically synthesized and micronized 8-PN to menopausal women. Signs of systemic estrogenic activity after a single oral dose were seen in women who received 750 mg 8-PN, confirming the *in vitro* about 70-fold lower estrogenic potency as compared to estradiol.

8-PN has shown its pure estrogenic properties in *in vitro* tests, animal models, and women who received 8-PN orally. However, whether the anti-ageing effects on the skin as known for e.g. estradiol would also be seen in topical application of 8-PN has so far not been elucidated.

Estrogens are known to have various positive effects on the structure and vitality of the skin. Estrogens increase or keep constant the amount and crosslinking of collagen and the degree of vascularisation of the skin, which is easily observed in estrogen-deficient states such as in the post-menopause. Further, the mitotic activity of the epidermis is stimulated by estrogens mediated by ER. Interestingly, the concentration of ER in the skin is not constant but differs between various areas of body skin, being highest in the facial epidermis. Both subtypes of ER are expressed in human skin, with ERß being the predominant subtype. ERs are mainly found in the stratum basale and stratum spinosum and to a lesser extent in the stratum granulosum, while the stratum corneum is free of it. In menopausal women not substituted with sex hormones, the collagen content of the skin decreases by 2% per year. The daily topical administration of estradiol leads to a marked increase in 4-hydroxy-proline, which is a marker for collagen synthesis. In addition, electron microscopic investigations of estradiol treated skin revealed morphologic improvements of the collagen fibres. The most potent natural estrogen is 17ß-estradiol (E2). It is clearly effective in treating age-related skin changes when administered as topical ointment. The same holds true for the less potent estrogen estradiol (E3) when applied as an ointment.

In conclusion, there is a large amount of evidence that estrogens directly applied to the skin prevent or treat age-related changes of the skin by increasing the amount and quality of collagen, stimulate cell mitosis and improve vascularisation. Thus, without any doubt, estrogens have positive effects on the structure, vitality, texture, smoothness and firmness of the skin. The only known route by which estrogenic effects are mediated is via the ERα or ERß. When ingested by the oral route of administration or by other routes (e.g. i.v., i.m., vaginal, rectal), ensuring systemic availability, estrogens are distributed via the blood stream and will reach ERs in the skin at a concentration that is dependent on dose and on the distribution volume of the active ingredient. However, using these treatment routes, the possible positive effect on the skin cannot be separated from unwanted systemic estrogenic effects. Further, when topically applying estrogens, the problem remains that active ingredients pass the skin, reach systemic circulation and act systemically. The actual transdermally absorbed dose for estradiol for example can be estimated by the treatment area and transdermal absorption rate which is about 130 ng/cm²/hour.

Whereas the preventive effects of estrogens on skin ageing have been well known for some time, the role of estrogens on hair growth and hair loss has been investigated only recently. For a long time, the obvious effects of androgens, e.g. androgenic alopecia (AGA), have covered the contribution of estrogens to hair growth homeostasis. Hair follicles possess an active sex hormone metabolism and have the ability to activate testosterone (T) to the much more active dihydro-testosterone (DHT) by the 5α-reductase, and to convert DHT into estradiol by aromatase. The estrogen-androgen balance can be disturbed by compromised enzyme and/or receptor activities which is thought to constitute the genetic contribution to male AGA and its typical local expression, as seen e.g. in forehead baldness. The balance can also be disturbed by estrogen deficiencies (e.g. hair loss in menopause, post partum, or during aromatase inhibitor treatment) or excess androgen production as in female hirsutism. Therefore, the local application of estrogens to increase hair growth in cases of estrogen deficiency is a scientifically sound principle if the systemic side effects of such treatments can be avoided. The same is true for conditions which are characterised by a preponderance of androgenic effects. Estrogen treatment then is able to support the locally missing estrogen and to inhibit the activation of T into DHT. Respective conditions are known as AGA, Seborrhea and Acne vulgaris.

Therefore, the technical problem underlying the present invention is to provide a topical composition having estrogenic activity in the skin but does not show any systemic estrogenic side effects, as well as uses thereof.

The solution to the above technical problem is achieved by the embodiments characterized in the claims.

In particular, the present invention relates to a topical composition comprising: a hop extract, and at least one member, selected from the group consisting of dermatologically and cosmetically acceptable carriers, excipients, and diluents; wherein the 8-prenyl naringenin (8-PN) content in the composition is between 0.1 and 1.0 % by weight.

The term "topical composition" as used herein refers to a composition that is topically applied to the skin in order to exert its specific cosmetic or therapeutic effects.

The amount of 8-PN in the topical composition of the present invention is 0.1 to 1.0 % by weight. More preferably, the composition comprises 8-PN in an amount of 0.3% to 0.7% by weight, most preferably 0.5% by weight.

Dermatologically and cosmetically acceptable carriers, excipients, and diluents that can be used in a topical composition according to the present invention are not particularly limited and are known to a person skilled in the art.

The topical composition of the present invention comprises a hop extract containing an effective amount of 8-PN. Preferably, the 8-PN content in the hop extract which in turn is then used in the topical composition of the present invention is between 1.0% and 10% by weight, preferably 5.0% to 10% by weight, more preferably 5.0% to 8.0% by weight.

The natural source of 8-PN is the hop plant. Worldwide there are several hundred hop varieties existing but only about 20 varieties are used as cultural plants and have economical importance. Discrimination can be made between bitter hops and aroma hops. The main varieties, as well as their 8-PN contents, are shown in Table 1.

**Table 1: Main hop varieties and respective 8-PN contents**

| **Aroma Hops** | | **Bitter Hops** | |
|---|---|---|---|
| **Variety** | **µg 8-PN/g** | **Variety** | **µg 8-PN/g** |
| Hallertau Tradition | 445 | Hallertau Northern Brewer | 253 |
| Hallertau Hersbrucker Spät | 447 | Hallertau Nugget | 438 |
| Hallertau Perle | 413 | Hallertau Target | 216 |
| Hallertau Spalter Select | 309 | Hallertau Magnum | 297 |
| Hallertauer Tradition | 445 | Hallertau Taurus | 189 |
| Slowenien Aurora | 373 | | |
| Tschechien Saazer | 476 | | |

| | | | |
|---|---|---|---|
| Concentrations of 8-PN (µg/g dry weight) given were analyzed in ethanol extract. | | | |

It is obvious that the content of 8-PN in hop is different in terms of the particular hop variety, extraction conditions and growth conditions of the hop plant, such as soil and weather. Therefore, the variance of the 8-PN content in ethanol extract produced from harvested hop varieties is large and ranges from approximately 200 to 500 µg/g dry weight. However, the use of hop extracts for cosmetic products requires a constant concentration of 8-PN in such pre-product extraction batches and also a constant ratio of 8-PN concentration to the concentration of other phenolic compounds such as XH and IXH.

Accordingly, in a preferred embodiment, the hop extract in the topical composition of the present invention is obtainable by a method, comprising the steps:
(a) providing an alcoholic hop extract,
(b) separating essential oils and bitter acids from the extract using supercritical CO₂ or CO_{2 fl},
(c) fractionating the residual with the aid of a suitable carrier and eluting with an organic solvent,
(d) treating the extract with alkaline for isomerization of 6-PN to 8-PN, and
(e) spiking the extract obtained in step (d) with 8-PN, in order to normalize to the desired 8-PN content, such that the 8-PN content in the hop extract is between 1.0% and 10% by weight.

Methods for providing alcoholic hop extracts, separating essential oils and bitter acids from the extract using supercritical CO₂ or CO_{2 fl}, fractionating the residual with the aid of a suitable carrier and eluting with an organic solvent, treating extracts with alkaline for isomerization of 6-PN to 8-PN, and spiking extracts with 8-PN, in order to normalize to the desired 8-PN content are not particularly limited and are known to a person skilled in the art.

In particular, methods for separating essential oils and bitter acids from a hop extract include for example mixing the hop extract with diatomaceous earth as carrier and re-extracting the extract with supercritical CO₂ or fluid CO₂ (CO_{2 fl}). Further, methods for absorbing the residual part to a suitable material and eluting it with an organic solvent include for example fractionation of the extract with the aid of polyvinylpolypyrrolidone and subsequent elution of the absorbed fraction with ethyl acetate. Furthermore, methods for subjecting the eluted extract to an alkaline treatment in order to isomerize 6-PN to 8-PN include for example isomerization in diluted NaOH solution (5% w/v).

Surprisingly, the penetration of 8-PN into the skin can be largely increased by specific C12-compounds (*i.e*. lauric acid or dodecanol) or esters or ethers of such compounds alone or in combination with propylene glycol (cf. Example 1). Accordingly, in a preferred embodiment, the topical composition of the present invention further comprises a penetration enhancer. Preferably, the penetration enhancer is selected from the group consisting of lauric acid and esters and ethers thereof, and dodecanol and esters and ethers thereof, and combinations thereof. In a preferred embodiment, the penetration enhancer further comprises propylene glycol. In another preferred embodiment, the penetration enhancer is contained in the topical composition of the present invention in an amount of 0.1 to 5% (w/w), more preferably 0.5 to 2% (w/w), most preferably 1% (w/w).

In a preferred embodiment of the topical composition of the present invention, said compositions do not contain estradiol, estriol or any derivatives thereof.

In further preferred embodiments, the topical composition of the present invention is for use in the prophylactic or therapeutic treatment of a condition, selected from the group consisting of androgenic Alopecia, Seborrhea and Acne vulgaris.

In a further aspect, the present invention relates to the use of the topical composition according to the present invention for providing increased hair growth, e.g. in estrogen deficiency conditions or androgen preponderance conditions, beneficial effects on androgenic Alopecia, Seborrhea and Acne vulgaris, e.g. in conditions of androgen preponderance, and/or at least one of the cosmetic skin care benefits, selected from the group consisting of positive effects on the structure, vitality, texture, smoothness, wrinkling, sagging, dryness, photodamage, firmness, collagen levels, and decorin levels of the skin. Said use comprises applying the topical compositions of the present invention to the skin. Preferably, the above cosmetic skin care benefits are due to a counteraction of the topical composition of the present invention to ageing effects of the skin which are caused by oxidative stress.

It has surprisingly been found that the dermal administration of 8-PN leads to very low transdermal absorption rates (cf. Example 2). In particular, only about 3 ng 8-PN/cm²/hour became transdermally absorbed as was determined by the renal excretion of 8-PN. This is an important piece of information because it is the basis for a risk assessment estimating the size of a possible systemic estrogenic effect. Calculating the maximum systemically available 8-PN dose as a worst case scenario, *i.e.* with a transdermal absorption rate of 5 ng/cm²/hour, sink conditions for the area dose and a treatment area of 1000 cm² (facial skin and neck), a daily systemic dose of about 120 µg 8-PN might reach the systemic circulation. This dose would correspond to a pharmacologically active dose of 1.7 µg E2, a dose without any measurable effect. In addition, the 8-PN dose of 120 µg is clearly within the range of the amounts generated by the drinking of 1 liter of beer spiced with natural hop. Up to 1 mg/d of 8-PN are estimated to burden the body of moderate beer drinkers. Even an up to 10-fold increase in percutaneous absorption by penetration enhancers (from worst case calculation of 5 to 50 ng/cm²/hour) would not burden the system with a pharmacologically active amount of 8-PN, since only about 1.2 mg 8-PN per day would become systemically available. This worst case scenario shows that there is no risk for a systemic estrogenic effect of 8-PN when applied as a topical formulation to the head, neck, face and décolleté skin area.

Accordingly, the use of the topical composition according to the present invention preferably does not affect systemic physiological processes by the estrogenic activity of 8-prenyl naringenin (8-PN). Further, said use preferably does not produce a higher transdermal flux than 50 ng 8-PN/cm²/h.

Surprisingly, the anti-oxidative effect of hop extracts produced by the method of the present invention is markedly higher than could be expected from the anti-oxidative power of the known ingredients (cf. Example 4). It is known that the anti-oxidative effects of 8-PN and XH are similar (10 and 8.3 mmol/g trolox-equivalents, respectively), while the potency of IX is somewhat lower (6.8 mmol/g trolox-equivalents). Remarkably, the anti-oxidative potency of the isomerized hop extract was found to be 3.5 fold higher than a hop extract containing mostly XH (cf. Example 4). The radical scavenger effect of 8-PN and the anti-oxidative properties of the hop extract together are highly effective in anti-aging, since skin ageing is thought to be at least in part due to oxidative stress. Further, cosmetics containing effective anti-oxidative ingredients will effectively decrease UVB-induced erythema and edema, and will decrease the number of sunburnt cells.

Examples for the preparation of topical compositions of the present invention are given in Example 5.

The figures show:
Figure 1:
   Structural formulas of xanthohumol **(1),** 6-prenyl naringenin **(2),** 8-prenyl naringenin **(3),** and iso-xanthohumol **(4).**
Figure 2:
   Transdermal flux of 8-PN (µg/cm²/h) in the "Franz-Chamber-Model" using skin of hairless mice (means ± standard deviation of three independent experiments).
Figure 3:
   Relative amounts of 8-PN (percent of dose applied) in different compartments 30 hours after application with various penetration enhancers or propylene glycol to hairless mouse skin in the Franz-Chamber-Model.
Figure 4:
   Daily urinary excretion of 8-PN before, during and after topical treatment with a cream containing 0.125% (w/w) 8-PN.

The present invention will now be further illustrated in the following examples without being limited thereto.

### Examples

### Example 1:

### Investigation of the penetration of 8-PN into the skin by use of the Franz-Chamber-Model

### Description of study

The aim of the study was to investigate the rate of percutaneous flux of 8-PN through living skin and to determine the distribution of 8-PN between different compartments at steady state conditions.

The model used is known as Franz-Chamber which is constructed by a heated outer cell and an inner flow cell through which a pre-warmed receptor fluid is pumped at a constant rate. The flow cell is sealed on top by hairless mouse skin excised shortly before the start of experiment. 8-PN was placed on the skin and the receptor fluid was collected in fractions. At the end of experiment the not absorbed drug was recovered, the skin removed and separated into stratum corneum and dermis. Analysis of 8-PN amounts was performed in receptor fluid, dermis, stratum corneum and in the not absorbed formulation.

Phosphate buffered saline (PBS) containing 1 mg/mL bovine serum albumin (BSA), 100 U/mL Penicillin (Roche), and 100 µg/mL Streptomycin (Roche) was used as receptor medium. Flow rate through the receptor chamber was 2 mL/min at 33° C.

Tritiated (³H) 8-PN was used (25µC/mg) at a concentration of 1mg/ml carrier solution. 20 µL of carrier solutions were applied to a 2 cm² skin area.

Five experimental formulations (carriers) were investigated:
● Propylene glycol (PG)
● Isopropyl myristate (IPM)
● Lauryl glycol / Transcutol (8:2 v/v)
● PG plus 10 % (v/w) lauric acid (LS)
● PG plus 10 % (v/v) lauryl alcohol (LA)

Experiments were run under non-occlusive conditions for 30 hours. Sample preparation and measurements were carried out according to routine methods of radioactivity analysis.

### Results

### Flux rate

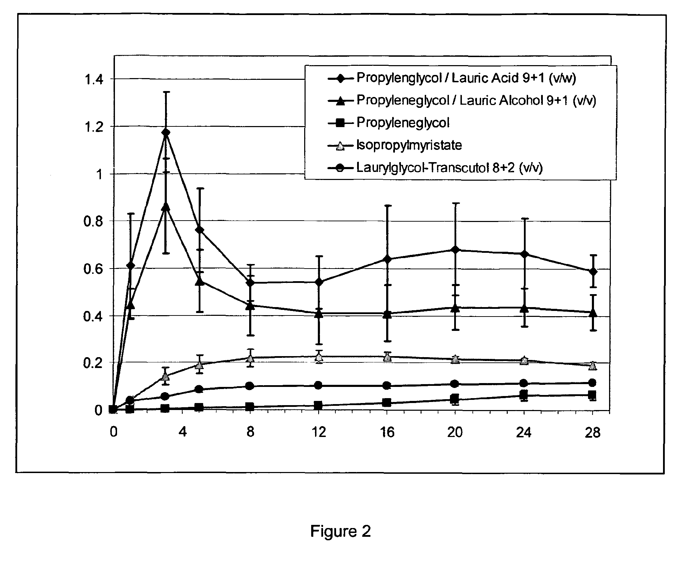

The calculated transdermal flux rates of 8-PN applied in various carriers are displayed in Figure 2. Obviously, the properties of carriers had a great influence on the permeation of 8-PN through the intact mouse skin.

Applied in the hydrophilic carrier propylene glycol, the transdermal flux was below 0.05 µg/cm²/h. When applied in the amphiphilic carrier Lauroglycol/Transcutol the flux reached a constant value of 0.1 µg/cm²/h. The highest flux of 0.21 µg/cm²/h was reached with the lipophilic carrier isopropyl myristate.

The addition of penetration enhancers changed extent and time course of 8-PN transdermal fluxes. PG was either mixed with 10 % LA or LS. Both penetration enhancers showed similar effects increasing the constant (steady state) transdermal fluxes to 0.45 or 0.65 µg/cm²/h for LA and LS, respectively, i.e. 5 to 6 fold when compared to the amphiphilic carrier.

### Distribution and mass balance

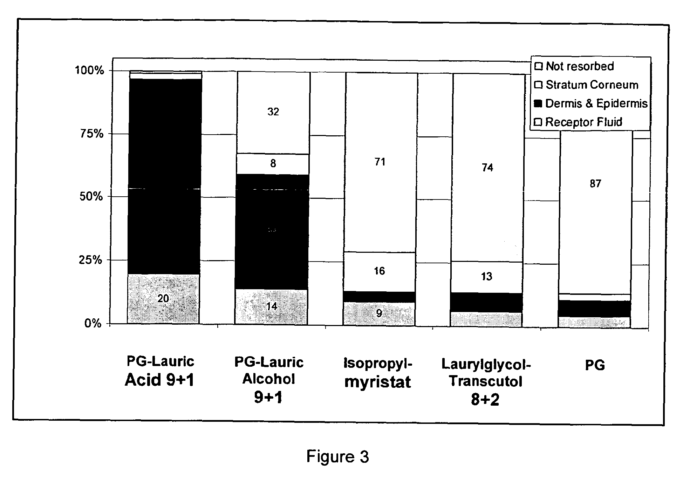

At the end of experiments a mass balance was calculated (Fig. 3). Total recoveries were between 75 and 95 % of administered dose. In order to directly compare the various experiments, results were normalized to individual recovery.

In agreement with the transdermal fluxes of 8-PN dissolved in PG, LG/TC, or IPM, 87, 74, and 71 % of doses were not absorbed but recovered from the skin surface. Surprisingly, substantial parts of the doses (13-16%) were found in *stratum corneum* in case of the amphiphilic or lipophilic carrier, but not in case of the hydrophilic PG. Low dose parts have passed the skin. Admixture of penetration enhancers changed the drug distribution. Although the observed flux rates were comparatively high in case of PG/LS or PG/LA only 14 (PG/LA) or 20 % of recovered doses (PG/LS) were found in the receptor fluid. Only minor dose parts were found not absorbed in case of PG/LS and about one third in case of PG/LA. While less than 10 % of doses were located in the stratum corneum barrier most of the drug (77% with PG/LS, 45 % with PG/LA) was found in the dermis (remaining skin).

### Conclusions

The present study on 8-PN using the Franz-Chamber model revealed:
● Lipophilicity of carriers increases percutaneous flux.
● Penetration enhancers increase the drug concentration in the dermis and might be helpful to reach target drug levels in the skin.
● Lauric acid (LS) was shown to be an efficient enhancer.
● The percutaneous flux of 8-PN is comparatively low excluding a systemic estrogenic effect after topical treatment.

### Example 2:

### Determination of the percutaneous flux of 8-PN and estimation of the daily dose taken up via the skin

### Description of study

The aim of the investigation was to calculate the extent of percutaneous absorption of 8-PN in man.

The treatment area was the face. The right half of the face was treated with verum cream (VC, 0.125% 8-PN w/w), the left half with placebo cream (PC). Treatment was twice a day (in the morning and in the evening) for 30 days (60 treatments in total). Urine was collected three days before, during and up to 5 days after the end of the treatment. Urine collection periods were 6 to 8 hours during day-time. Frequency of urine collection was every 1 to 3 days. Volumes were recorded, an aliquot taken and kept frozen until analysis.

The participant (smoking male, 60 yrs, 84 kg, 182 cm) did not drink beer two weeks before the tests and during the test period of 5 weeks. No other restrictions were found to be necessary.

### Drug preparation

50 mg 8-PN (GMP batch) were incorporated into 40 g of a cream (Nivea Visage^{®}, moisture spending daytime cream with provitamin B5 and jojobaoil for normal and mixed skin, batch 34650054M) resulting in a 0.125% cream. Incorporation of 8-PN was achieved by evaporation of 2.5 ml of an alcoholic solution (20 mg 8-PN/ml) and by dissolving the amorphic residues in increasing amounts of the cream under stirring. After 24 hours the cream was transferred into plastic tubes. Tubes were screwed and kept at ambient temperature during trial time.

### Sample analyses and evaluations

The calculation of the daily dose and the area dose was achieved by the determination of totally used cream (weight on day 0 minus weight on day 30) and the estimation of the treatment area (225 cm²). The area dose was 1.25 µg/cm² and the total daily dose about 560 µg 8-PN/d.

Defined aliquots of all 20 urine samples were hydrolyzed enzymatically (glucuronidase/arylsulphatase) and then extracted with tert. butyl methyl ether. Extracts were evaporated (N₂) and residues dissolved in assay buffer. Each sample was analyzed in duplicate (duplicate extraction) by means of a specific radioimmunoassay (RIA) known in the art. Results from RIA (ng/ml) were converted into the amount of 8-PN in each sample (µg). Numbers were divided by the time of collection period (µg/h) and then multiplied by 24 to give the daily excretion of 8-PN conjugates (µg/d). The mean excretion rate per day was calculated and related to the percent of a 25 mg dose, the same volunteer had orally absorbed in an earlier study, *i.e.* 14%. Thus, the amount of 8-PN in the urine after dermal treatment was regarded to represent 14 % of the actually transdermally absorbed dose.

### Results

Study performance, adverse effects:
No subjective systemic or local side effects were recorded. No skin irritations were observed.

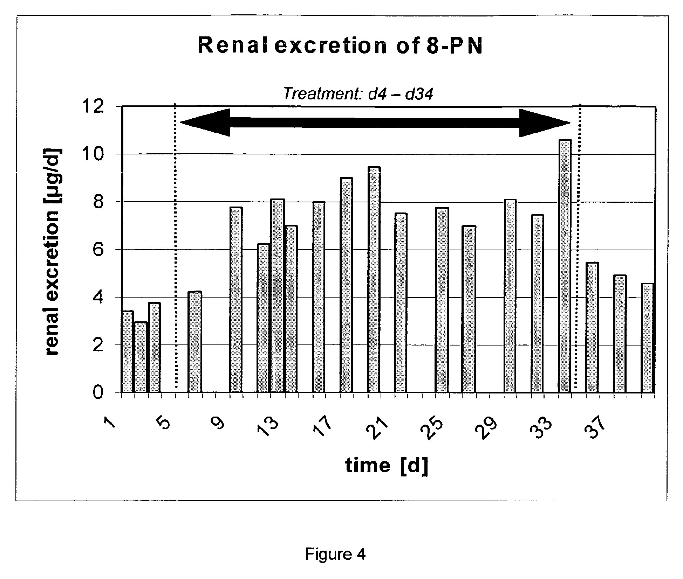

Renal excretion of 8-PN conjugates:
Before start of treatment an average 3.38 µg 8-PN of unknown origin were excreted. After start of treatment this amount increased to 6 to 10 µg/d (cf. Figure 4). On an average at steady state (days 9 through day 34) 8.0 ± 1.14 µg 8-PN were excreted daily. Corrected for blank values a daily excretion of 4.6 µg can be assigned to topical treatment. After the end of treatment 8-PN excretion slowly approached the pre-treatment blank values.

### Discussion

The present results allow a detailed estimation of the percutaneous flux of 8-PN. Estimation is based upon the renal excretion of 14% of an oral 25 mg dose, which was found in an earlier study in the same volunteer. A systemically available dose of 33 µg (7.14 x 4.62 µg) is calculated. This is equivalent to a percutaneous absorption of 5.9% of the daily topical dose formulated in a conventional cream (Nivea Visage®). Calculated from the estimated daily dose of 33 µg 8-PN and the treatment area, a percutaneous flux of 2.9 ng/cm²/h results. This number is more than 40 times lower than the flux of E₂ (about 130 ng/cm²/h) applied as cream or patch.

Concerning a visible anti-ageing effect of treatment, results from interviews performed during the time period of 20 to 30 days of treatment can be mentioned. Nine independent persons were asked to inspect the total face and then to estimate which part had been treated with an anti-ageing cream. Seven of nine decided for the right (VC treated) half of the facial skin.

### Example 3:

### Extraction method of hop and normalization of 8-PN content in extracts from different hop varieties

The basis of extract was natural hops. Dried bitter or aroma hops were extracted with fermentation ethanol (90% v/v). The extract contained all bitter acids and prenylflavonoids and almost all hop essential oils. It was a green viscous fluid containing less than 0.3% ethanol. Depending on hop variety and crop year it contained 8-PN in a concentration of up to 500 µg/g dry weight.

This extract was then mixed with Diatomaceous Earth (DE) as carrier and reextracted with supercritical CO₂ or CO_{2 fl} to separate the essential oil and bitter (α and ß) acids from the residue. The remaining residue on the DE carrier contained less than 0.1% beta acids, up to 2% iso-alpha acids, up to 1% alpha acids, up to 3% XH and less than 0.05% 8-PN. The residue was a green powder having a density of 550 g/L.

The powder was taken up in ethanol, freed from DE and the solution was then pumped through polyvinylpolypyrrolidone (PVPP). Elution of the absorbed extract with ethyl acetate resulted in a yellow powder of a density of 500 g/L containing up to 85% XH, 0.5% 8-PN and 2% 6-PN.

Alkaline treatment with diluted NaOH solution (5% w/v) in part isomerized XH to IXH and 6-PN to 8-PN. The result was a hop extract containing up to 1.5% 8-PN. The ratio c(XH)/c(8-PN) was approximately 2 and the ratio c(IXH)/c(8-PN) approximately 40. The extract was then spiked with a 10% alcoholic solution of authentic 8-PN for normalization.

### Example 4:

### Determination of anti-oxidative effects of hop extracts by means of TEAC-assay

### Principle of assay

The TEAC-assay (Trolox® Equivalent Antioxidative Capacity) is able to measure the anti-oxidative potency of a solution by means of an absorption measurement. The reference is Trolox, a vitamin E derivative. The oxidative milieu is created by potassium persulfate, which generates a green colored radical cation of ABTS, i.e. 2,2'-azinobis-3-ethyl benzthiazoline-6-sulfonic acid. Anti-oxidants will slow down the reaction followed at 734 nm photometrically. Results are expressed as mmol Trolox/L or g weight.

### Sample preparation

Two hop extracts were used, *i.e.* Xanthoflav (XHF) and Isoxanthoflav (IXF), characterized by the following contents:

| | XHF | IXF |
|---|---|---|
| Xanthohumol | 75.0 | 2.0 |
| Isoxanthohumol | 1.0 | 73.0 |
| 8-PN | 0.5 | 4.0 |
| 6-PN | 1.0 | 0.5 |

| | | |
|---|---|---|
| Values are given in % of weight. | | |

Triplicates of 1 to 2 mg per extract were dissolved in methanol/DMSO (1:1 v/v) and subsequently diluted with PBS buffer (1:50 or 1:100).

### Results

The anti-oxidative potency was (means ± standard deviation of 3 determinations):

| | |
|---|---|
| XHF | 3.66 ± 0.13 mmol/g trolox-equivalents |
| IXF | 12.65 ± 0.24 mmol/g trolox-equivalents |
| Xanthohumol | 4.22 ± 0.02 mmol/g trolox-equivalents |
| 8-PN | 12.33 ± 0.17 mmol/g trolox-equivalents. |

### Example 5:

### Compositions according to the present invention

In the following, eight different formulations according to the present invention are described that are suitable for the uses according to the present invention. Preferably, formulations are marketed in one way packings. The percentages indicated are by weight of the composition.

### 5.1 Preparation of an o/w emulsion cream (Cremor basalis)

| **Ingredient** | **Weight %** |
|---|---|
| Hop extract | 5.0 |
| Glycerolmonostearate 60 | 4.0 |
| Cetylalcohol | 6.0 |
| Triglyglyceride, C12-C18 | 7.5 |
| Vaseline, white | 23.0 |
| Macrogol-20-glycerolmonostearate | 7.0 |
| Propylenglycol | 9.0 |
| Butylated hydroxy toluene | 0.01 |
| Water, purified | ad 100.0 |

### 5.2 Preparation of a hydrophilic basic emulsion (Emulsio basalis hydrophilica)

| **Ingredient** | **Weight %** |
|---|---|
| Hop extract | 5.0 |
| Sorbitolmonostearate, type I | 2.0 |
| Macrogol-8-stearate, type I | 2.0 |
| Triglyglyceride, C12-C18 | 5.0 |
| Glycerine 85% | 5.0 |
| Citrate acid, water free | 0.07 |
| Potassium sorbate | 0.14 |
| Water, purified | ad 100.0 |

### 5.3 Preparation of an anionic hydrophilic creme (Cremor anionicus hydrophilicus SR)

| **Ingredient** | **Weight %** |
|---|---|
| Hop extract | 5.0 |
| Cetyl stearyl alcohol, type A | 21.0 |
| 2-Ethylhexyl lauromyristate | 10.0 |
| Glycerine 85% | 5.0 |
| Citrate acid, water free | 0.07 |
| Potassium sorbate | 0.14 |
| Water, purified | ad 100.0 |

### 5.4 Preparation of a non-ionic hydrophilic creme (Cremor nonionicus hydrophilicus SR)

| **Ingredient** | **Weight %** |
|---|---|
| Hop extract | 5.0 |
| Non-ionic emulsifying alcohols | 21.0 |
| 2-Ethylhexyl lauromyristate | 10.0 |
| Glycerine 85% | 5.0 |
| Citrate acid, water free | 0.07 |
| Potassium sorbate | 0.14 |
| Water, purified | ad 100.0 |

### 5.5 Preparation of a hydrophilic emulsion

| **Ingredient** | **Weight %** |
|---|---|
| Hop extract | 5.0 |
| Sorbitolmonostearate, type I | 2.0 |
| Macrogol-8-stearate, type I | 2.0 |
| Triglyglyceride, C12-C18 | 5.0 |
| Glycerine 85% | 5.0 |
| Propylene glycol:lauric acid (9:1 v/v) | 1.0 |
| Citrate acid, water free | 0.07 |
| Potassium sorbate | 0.14 |
| Water, purified | ad 100.0 |

### 5.6 Preparation of a non-ionic creme

| **Ingredient** | **Weight %** |
|---|---|
| Hop extract | 5.0 |
| Non-ionic emulsifying alcohols | 21.0 |
| 2-Ethylhexyl lauromyristate | 10.0 |
| Glycerine 85% | 5.0 |
| Propylene glycole monolaureate | 1.0 |
| Citrate acid, water free | 0.07 |
| Potassium sorbate | 0.14 |
| Water, purified | ad 100.0 |

### 5.7 Preparation of an ethanolic tincture

| **Ingredient** | **Weight %** |
|---|---|
| Hop extract | 5.0 |
| Ethanol | 70.0 |
| Propylene glycole monolaureate | 1.0 |
| Isopropyl myristate | 1.0 |
| Water, purified | ad 100.0 |

### 5.8 Preparation of an alcoholic tincture

| **Ingredient** | **Weight %** |
|---|---|
| 8-PN | 0.5 |
| Isopropanol | 70.0 |
| Propylene glycole monolaureate | 1.0 |
| Isopropyl myristate | 1.0 |
| Water, purified | ad 100.0 |

## Claims

1. A topical composition comprising:
a hop extract, and at least one member, selected from the group consisting of dermatologically and cosmetically acceptable carriers, excipients, and diluents;
wherein the 8-prenyl naringenin (8-PN) content in the composition is between 0.1 and 1.0 % by weight.

2. The topical composition according to claim 1, wherein the hop extract is obtainable by a method, comprising the steps:
(a) providing an alcoholic hop extract,
(b) separating essential oils and bitter acids from the extract using supercritical CO₂ or CO_{2 fl},
(c) fractionating the residual with the aid of a suitable carrier and eluting with an organic solvent,
(d) treating the extract with alkaline for isomerization of 6-PN to 8-PN, and
(e) spiking the extract obtained in step (d) with 8-PN, in order to normalize to the desired 8-PN content, such that the 8-PN content in the hop extract is between 1.0% and 10% by weight.

3. The topical composition according to claim 1 or claim 2, further comprising a penetration enhancer, selected from the group consisting of lauric acid and esters and ethers thereof, dodecanol and esters and ethers thereof, and combinations thereof.

4. The topical composition according to claim 3, wherein said penetration enhancer further comprises propylene glycol.

5. The topical composition according to any one of claims 1 to 4, wherein the composition does not contain estradiol, estriol, or derivatives thereof.

6. The topical composition according to any one of claims 1 to 5 for use in the prophylactic or therapeutic treatment of a condition selected from the group consisting of andronergic Alopecia, Seborrhea and Acne vulgaris.

7. Use of the topical composition according to any one of claims 1 to 5 for providing at least one effect, selected from increased hair growth and cosmetic skin care benefits, selected from the group consisting of positive effects on the structure, vitality, texture, smoothness, wrinkling, sagging, dryness, photodamage, firmness, collagen levels, and decorin levels of the skin.

8. Use according to claim 7, wherein the cosmetic skin care benefits are due to counteracting ageing effects of the skin caused by oxidative stress.

9. Use according to claim 7 or 8, comprising applying said topical composition to the skin.

10. Use according to any one of claims 7 to 9, which does not affect systemic physiological processes by the estrogenic activity of 8-prenyl naringenin (8-PN).

11. Use according to any one of claims 7 to 10, which does not produce a higher transdermal flux than 50 ng 8-PN/cm²/h.
